# EUROPEAN PATENT APPLICATION

(11) **EP 4 541 358 A1**
(43) Date of publication of application: **23.04.2025**
(21) Application number: 23827030.0
(22) Date of filing: 12.06.2023
(51) Int. Cl.: A61K 35/32, A61K 9/14, A61K 31/7105, A61K 35/28, A61K 47/46, A61K 48/00, A61P 9/00, A61P 9/08, A61P 9/10, A61P 11/00, A61P 29/00, A61P 31/04, A61P 37/06, A61P 43/00

(54) **HMGB1 EXPRESSION SUPPRESSOR, PROPHYLACTIC OR THERAPEUTIC DRUG FOR ACUTE LUNG INJURY, ACUTE RESPIRATORY DISTRESS SYNDROME OR SEPSIS, AND METHOD FOR AMELIORATING SUCH DISEASE**

(30) Priority: 20.06.2022 JP 2022098639; 20.05.2023 JP 2023083544
(71) Applicant: Dexon Pharmaceuticals Inc., Tokyo, 103-0027 (JP)
(72) Inventor: KOGA Shoji, Tokyo 102-0082 (JP)
(74) Representative: Godemeyer Blum Lenze Patentanwälte Partnerschaft mbB - werkpatent
(86) International application number: PCT/JP2023/021640
(87) International publication number: WO 2023/248844

(57) **Abstract**

Provided are: a novel HMGB1 expression regulator including small extracellular vesicles which include a miRNA targeting a gene involved in HMGB1 expression and can regulate HMGB1 expression using the miRNA derived from the small extracellular vesicles; a prophylactic agent or therapeutic agent for acute lung injury, acute respiratory distress syndrome, or sepsis; and a method of ameliorating acute lung injury, acute respiratory distress syndrome, or sepsis. The small extracellular vesicle is preferably exosomes.

## Description

### Technical Field

The present invention relates to an HMGB1 expression regulator; a prophylactic agent or therapeutic agent for acute lung injury, acute respiratory distress syndrome, or sepsis; and a method of ameliorating acute lung injury, acute respiratory distress syndrome, or sepsis.

### Background Art

High-Mobility Group Box 1 (HMGB1) protein is released outside cells upon necrosis of any nucleated cells and also through activation of normal functions in living cells in a case of release from dendritic cells, macrophages, and the like. Although most of HMGB1 usually accumulates in nuclei, HMGB1 is released into cell cytoplasm when cells are subjected to lipopolysaccharide stimulation or bacterial infection, and is released outside cells during an inflammatory response or infection.

Studies on microRNAs (miRNAs) have progressed, and functions of miRNAs related to HMGB1 and target genes have been known (see NPLs 1 and 2).

NPL 1 indicates that miR-142-3p inhibits chondrocyte apoptosis and inflammation in osteoarthritis by inhibiting the HMGB 1-mediated NF-kB signaling pathway.

NPL 2 indicates that miR-22-3p expression is negatively correlated with HMGB 1 expression in human arteriosclerosis obliterans (ASO) tissues, that miR-22-3p is a key molecule in regulating human artery vascular smooth muscle cell (HASMC) proliferation and migration by targeting HMGB1, and that miR-22-3p and HMGB1 are therapeutic targets in treatment of human arteriosclerosis obliterans (ASO).

Here, HMGB1 is known to be involved in acute lung injury and sepsis. For example, NPL 3 indicates that HMGB1 is a late inflammatory mediator associated with sepsis, malignant tumor, and immunological diseases, and that HMGB1 and autophagy are related to pathogenesis of many pneumonic diseases including acute lung injury (ALI).

On the other hand, isolated exosomes are known to be involved in acute lung injury, acute respiratory distress syndrome (ARDS), or sepsis. For example, PTL 1 describes an isolated exosome (i) including one or more markers selected from the group consisting of ALIX, TSG101, TGFBR2, SMAD1, SMAD2, SMAD3, SMAD5, and CD105; and/or (ii) not including one or more markers selected from the group consisting of FLOT1, CD9, CD81, CAV1, EGFR, AKT1, and AKT2.

### Citation List

### Patent Literature

PTL 1: JP2021-020908A

### Non Patent Literature

NPL 1: inflammation, (2016)39, 1718-1728
NPL 2: Cell. Physiol. Biochem., (2017)42, 2492-2506
NPL 3: Med Sci Monit, (2019)25: 1828-1837

### Summary of Invention

### Technical Problem

PTL 1 does not disclose HMGB1.

Although NPLs 1 and 2 describe the functions and the target genes of miRNAs related to HMGB1 in vasculitis, NPLs 1 and 2 do not describe administration of these miRNAs as a therapeutic agent for acute lung injury, acute respiratory distress syndrome, or sepsis.

Although NPL 3 describes the association between HMGB1 and acute lung injury, acute respiratory distress syndrome, or sepsis, NPL 3 does not describe association with small extracellular vesicles (sEVs) or miRNAs.

A problem to be solved by the present invention is to provide a novel HMGB1 expression regulator capable of regulating HMGB1 expression using a miRNA derived from small extracellular vesicles.

### Solution to Problem

The present inventors have found that small extracellular vesicles including a specific microRNA can regulate (suppress, inhibit, or the like) expression of HMGB1 protein and/or HMGB1 gene (Hmgb1).

Specifically, the present invention and preferrable configurations of the present invention are as follows.
[1] An HMGB1 expression regulator,
   wherein the HMGB1 expression regulator includes small extracellular vesicles, and
   the small extracellular vesicles include a miRNA targeting a gene involved in HMGB1 expression.
[2] The HMGB1 expression regulator according to [1], in which
   the small extracellular vesicles are exosomes.
[3] The HMGB1 expression regulator according to [1], in which
   the miRNA targeting a gene involved in HMGB1 expression includes at least one miRNA in the following HMGB1-related miRNA group:
   hsa-let-7b-5p, hsa-let-7e-5p, hsa-let-7g-5p, hsa-miR-100-5p, hsa-miR-103a-3p, hsa-miR-106b-5p, hsa-miR-107, hsa-miR-1179, hsa-miR-1183, hsa-miR-1236-3p, hsa-miR-1237-3p, hsa-miR-1247-3p, hsa-miR-129-5p, hsa-miR-1304-3p, hsa-miR-1307-3p, hsa-miR-141-3p, hsa-miR-142-3p, hsa-miR-142-5p, hsa-miR-145-5p, hsa-miR-148a-3p, hsa-miR-148b-3p, hsa-miR-150-5p, hsa-miR-17-5p, hsa-miR-181d-5p, hsa-miR-186-3p, hsa-miR-186-5p, hsa-miR-18a-3p, hsa-miR-1913, hsa-miR-193b-3p, hsa-miR-1976, hsa-miR-204-5p, hsa-miR-205-5p, hsa-miR-206, hsa-miR-20a-5p, hsa-miR-20b-5p, hsa-miR-211-5p, hsa-miR-212-5p, hsa-miR-218-5p, hsa-miR-22-3p, hsa-miR-24-3p, hsa-miR-26b-5p, hsa-miR-300, hsa-miR-302c-3p, hsa-miR-324-3p, hsa-miR-328-3p, hsa-miR-329-3p, hsa-miR-340-5p, hsa-miR-34a-5p, hsa-miR-362-3p, hsa-miR-373-3p, hsa-miR-381-3p, hsa-miR-410-3p, hsa-miR-4275, hsa-miR-4284, hsa-miR-4324, hsa-miR-4460, hsa-miR-4532, hsa-miR-4638-5p, hsa-miR-4673, hsa-miR-4691-5p, hsa-miR-4707-3p, hsa-miR-4727-3p, hsa-miR-4793-5p, hsa-miR-495-3p, hsa-miR-505-3p, hsa-miR-512-3p, hsa-miR-5193, hsa-miR-5196-3p, hsa-miR-520a-3p, hsa-miR-520d-3p, hsa-miR-520h, hsa-miR-539-3p, hsa-miR-582-5p, hsa-miR-652-3p, hsa-miR-660-3p, hsa-miR-665, hsa-miR-92a-3p, and hsa-miR-93-5p.
[4] The HMGB1 expression regulator according to [1], in which
   the miRNA targeting a gene involved in HMGB1 expression includes at least one of hsa-let-7b-5p, hsa-miR-100-5p, hsa-miR-129-5p, hsa-miR-142-3p, hsa-miR-22-3p, hsa-miR-34a-5p, and hsa-miR-92a-3p.
[5] The HMGB1 expression regulator according to [1], in which
   the miRNA targeting a gene involved in HMGB1 expression includes at least hsa-let-7b-5p.
[6] The HMGB1 expression regulator according to [1], in which
   the small extracellular vesicles include the miRNA targeting a gene involved in HMGB1 expression at a concentration higher than a culture supernatant of dental pulp-derived stem cells.
[7] The HMGB1 expression regulator according to [1], in which
   the small extracellular vesicles are isolated through purification of a culture supernatant of dental pulp-derived stem cells, and
   the small extracellular vesicles do not include a component in the culture supernatant of dental pulp-derived stem cells excluding exosomes.
[8] A prophylactic agent or therapeutic agent for acute lung injury, acute respiratory distress syndrome, or sepsis, including, as an active ingredient, the HMGB1 expression regulator according to [1].
[9] A method of ameliorating acute lung injury, acute respiratory distress syndrome, or sepsis, the method including administrating an effective amount of the HMGB1 expression regulator according to [1] or an effective amount of the prophylactic agent or therapeutic agent for acute lung injury, acute respiratory distress syndrome, or sepsis according to [8] to a subject developing acute lung injury, acute respiratory distress syndrome, or sepsis.

### Advantageous Effects of Invention

According to the present invention, a novel HMGB1 expression regulator capable of regulating HMGB1 expression using a miRNA derived from small extracellular vesicles can be provided.

### Brief Description of Drawings

[FIG. 1] FIG. 1 is a heat map showing expression levels of miRNAs expressed on exosomes (HMGB1 expression regulator in Example 1) purified from a culture supernatant of dental pulp-derived stem cells targeting a gene involved in HMGB1 expression.
[FIG. 2] FIG. 2 is a graph of HMGB1 gene expression levels quantified in examples.

### Description of Embodiments

Hereinafter, the present invention will be described in detail. The description of the constituents described below may be made based on a representative embodiment or specific examples, but the present invention is not limited to such an embodiment. In the present specification, a numerical range expressed by using "to" means a range including the numerical values described before and after "to" as a lower limit value and an upper limit value.

### HMGB1 expression regulator

An HMGB1 expression regulator of the present invention includes small extracellular vesicles that include a miRNA targeting a gene involved in HMGB1 expression.

The HMGB1 expression regulator of the present invention can regulate HMGB1 expression using a miRNA derived from small extracellular vesicles. As a result, the HMGB1 expression regulator of the present invention is preferably capable of ameliorating acute lung injury, acute respiratory distress syndrome, or sepsis and is more preferably capable of preventing or treating acute lung injury, acute respiratory distress syndrome, or sepsis.

Hereinafter, a preferable embodiment of the HMGB1 expression regulator of the present invention will be described.

### Acute lung injury, acute respiratory distress syndrome, and sepsis

Acute lung injury (ALI) and acute respiratory distress syndrome (ARDS) refer to conditions in which sudden shortness of breath or respiratory distress occurs during the course of sepsis or pneumonia, or after aspiration, multiple trauma (a state where multiple areas of the body are injured), or the like, and a shadow (infiltrative shadow) is observed in both lungs on a chest X-ray radiograph. The arterial oxygen partial pressure is decreased (hypoxemia), and the conditions are called as either acute lung injury or acute respiratory distress syndrome depending on the severity.

Sepsis is a condition in which bacteria and the like enter the blood and proliferate due to, for example, infection by a virus or bacterium. Sepsis may lead to progression of organ damage and to a fatal outcome. No highly effective treatment method for sepsis has been established.

### Small extracellular vesicles

The HMGB1 expression regulator of the present invention includes small extracellular vesicles.

In the present invention, the small extracellular vesicles include a miRNA targeting a gene involved in HMGB1 expression.

The small extracellular vesicles are derived from dental pulp-derived stem cells or the like through secretion, budding, dispersion, or the like from mesenchymal stem cells such as dental pulp-derived stem cells, for example, and the small extracellular vesicles leach, are released, or are shed into a cell culture medium. The small extracellular vesicles are preferably included in a culture supernatant of dental pulp-derived stem cells or the like, and are more preferably small extracellular vesicles derived from a culture supernatant of dental pulp-derived stem cells. However, the small extracellular vesicles derived from the culture supernatant of dental pulp-derived stem cells do not necessarily need to be obtained from a culture supernatant of dental pulp-derived stem cells. For example, even in a case where small extracellular vesicles inside dental pulp-derived stem cells are isolated by an arbitrary method, the small extracellular vesicles can be regarded as the small extracellular vesicles derived from a culture supernatant of dental pulp-derived stem cells as long as the small extracellular vesicles are identical to the small extracellular vesicles that can be isolated from a culture supernatant of dental pulp-derived stem cells.

The small extracellular vesicles derived from a culture supernatant of dental pulp-derived stem cells or the like may be used in a state where the small extracellular vesicles are included in the culture supernatant, or may be used in a state where the small extracellular vesicles are purified from the culture supernatant. The small extracellular vesicles are preferably purified from a culture supernatant.

The origine of the small extracellular vesicles can be determined by a known method. For example, which stem cells, such as dental pulp-derived stem cells, adipose-derived stem cells, bone marrow-derived stem cells, or umbilical cord-derived stem cells, the small extracellular vesicles originate from can be determined by the method described in J Stem Cell Res Ther (2018)8:2. Specifically, the origin of each small extracellular vesicle can be determined based on the miRNA pattern of the small extracellular vesicle.

### miRNA

In the present invention, the small extracellular vesicles include a miRNA targeting a gene involved in HMGB1 expression.

In the present invention, miRNAs (or microRNAs) are RNA molecules having 21 to 25 bases (nucleotides), for example. The miRNA can regulate gene expression by suppressing decomposition of a target mRNA gene (target) or transcription process thereof.

In the present invention, the miRNA may be a single stranded miRNA (monomer) or a double stranded miRNA (dimer), for example. In addition, in the present invention, the miRNA is preferably a mature miRNA cleaved by a ribonuclease such as Dicer.

Sequences of miRNAs such as hsa-let-7b-5p described in the present specification are registered in known databases (for example, the miRBase database) in association with accession numbers, and those skilled in the art can unambiguously determine the sequences. For example, the accession number of hsa-let-7b-5p is MIMAT0000063, and the sequence thereof is registered in the miRBase database. Hereinafter, the accession number of each miRNA is omitted.

However, the miRNA herein includes a variant of the mature miRNA such as hsa-let-7b-5p differing by approximately 1 to 5 bases. In addition, each miRNA herein includes a polynucleotide which includes a base sequence having identity with the base sequence of each miRNA (for example, hsa-let-7b-5p) or a polynucleotide which includes a complementary base sequence thereof and has the same function as the miRNA of the present invention. "Identity" refers to the degree of sameness between sequences to be compared when the sequences are appropriately aligned and means the percentage (%) of exact matches of amino acids between the sequences. The sequences can be aligned using any algorithm such as BLAST, for example. The identity is, for example, 80%, 85%, 90%, 91%, 92%, 93%, 94%, 95%, 96%, 97%, 98%, or about 99%. The polynucleotide composed of a base sequence having identity may have, for example, a point mutation, a deletion, and/or an addition in the base sequence of the miRNA. The number of bases involved in the point mutation and the like is, for example, 1 to 5, 1 to 3, 1 or 2, or 1. The polynucleotide composed of a complementary base sequence is, for example, a polynucleotide hybridized with a polynucleotide composed of the base sequence of the miRNA under stringent conditions and includes a polynucleotide having the function of the miRNA in the present invention. The stringent conditions are not particularly limited but include the conditions described in paragraph [0028] of JP2017-184642A the content of which is incorporated herein by reference, for example.

In the present invention, the small extracellular vesicles preferably include the miRNA targeting a gene involved in HMGB1 expression at a concentration higher than that in the culture supernatant of dental pulp-derived stem cells. Hereinafter, a preferable mode of the miRNA included in the small extracellular vesicles is described.

### miRNA targeting a gene involved in HMGB1 expression

HMGB1 is released outside cells upon necrosis of any nucleated cells and also through activation of normal functions in living cells in a case of released from dendritic cells, macrophages, and the like. It is considered that HMGB1 liberated to the outside of cells induces a biological defense response such as innate immune or hemostasis in a localized and transient situation and also plays an important role in repair. However, when HMGB1 exerts systemic effects, HMGB1 acts as a mediator of shock and disseminated intravascular coagulation (DIC).

HMGB1 is mainly present in nuclei and has been identified as a protein playing a role in stabilizing chromatin structure and gene transcription reaction. A portion of HMGB1 is also present in cell cytoplasm and is involved in recognizing a nucleic acid taken up from the outside of cells and inducing autophagy. Furthermore, HMGB1 is released outside cells from nuclei in response to an inflammatory stimulus such as that from a lipopolysaccharide or in response to cell death. In particular, since HMGB1 released outside cells is recognized by an innate immune receptor including a toll-like receptor, HMGB1 is involved in sepsis, autoimmune diseases, and inflammation during ischemia-reperfusion injury and organ transplantation. Indeed, administration of a neutralizing antibody against HMGB1 can ameliorate these clinical conditions. Cytoplasmic HMGB1 is important for suppressing sepsis induced by a lipopolysaccharide (LPS) and listerial infection (PNAS(2013) vol. 110, no. 51, 20699-20704).

Here, miR-142-3p inhibits chondrocyte apoptosis and inflammation in osteoarthritis by inhibiting the HMGB1-mediated NF-kB signaling pathway (inflammation, (2016)39, 1718-1728).

Expression of miR-22-3p negatively correlates with HMGB1 expression in human arteriosclerosis obliterans (ASO) tissues, miR-22-3p is an important molecule that targets HMGB1 and regulates human aortic smooth muscle cell (HASMC) proliferation and migration, and miR-22-3p and HMGB1 are therapeutic targets in treating human arteriosclerosis obliterans (ASO) (Cell. Physiol. Biochem., (2017)42, 2492-2506).

HMGB1, which is an inflammatory mediator, directly targets miR-129-5p, and miR-129-5p suppresses apoptosis and inflammatory responses via the HMGB1//TLR4/NF-κB pathway (Biosci Rep. (2020)40(3)).

On the other hand, NPL 3 (Med Sci Monit, (2019)25: 1828-1837) indicates that HMGB1 is a late inflammatory mediator associated with sepsis, malignant tumor, and immunological diseases, and that HMGB1 and autophagy are related to pathogenesis of many pneumonic diseases including acute lung injury (ALI). Therefore, the HMGB1 expression inhibitor can be used as a prophylactic agent or therapeutic agent for acute lung injury or acute respiratory distress syndrome.

Sepsis begins when a pathogen invades the body, and systemic inflammation is triggered. This inflammatory response involves a "keyhole (RAGE) in which a glycated protein called AGE fits." Invasion of a pathogen induces production of HMGB1, which stimulates the RAGE and progresses sepsis. That is, when the body is infected by a pathogen or a virus, HMGB1 produced in the body binds to the keyhole (RAGE), leading to an onset of sepsis. A substance called a RAGE aptamer acts as a lid for the RAGE and covers the keyhole to inhibit binding of HMGB1 and the RAGE, enabling suppression of death from sepsis in an animal (Oxidative Medicine and Cellular Longevity, (2021) Article ID 9932311).

Release of HMGB1 is strongly suppressed by plasma histidine-rich glycoprotein (HRG), and excessive production of inflammatory cytokines is suppressed. The vascular endothelial cell protection effect of HRG may lead to development of sepsis therapeutic methods (J. isci. (2020) Volume 23, Issue 6, 101180).

Therefore, an HMGB1 expression inhibitor can be used as a prophylactic agent or therapeutic agent for sepsis.

As described above, miRNAs targeting a gene involved in HMGB1 expression (HMGB1-related miRNA group described below) are also effective as a therapeutic agent for vasculitis, and therefore, are also effective as a prophylactic agent or therapeutic agent for acute lung injury, acute respiratory distress syndrome, or sepsis. HMGB1-related miRNA group:
hsa-let-7b-5p, hsa-let-7e-5p, hsa-let-7g-5p, hsa-miR-100-5p, hsa-miR-103a-3p, hsa-miR-106b-5p, hsa-miR-107, hsa-miR-1179, hsa-miR-1183, hsa-miR-1236-3p, hsa-miR-1237-3p, hsa-miR-1247-3p, hsa-miR-129-5p, hsa-miR-1304-3p, hsa-miR-1307-3p, hsa-miR-141-3p, hsa-miR-142-3p, hsa-miR-142-5p, hsa-miR-145-5p, hsa-miR-148a-3p, hsa-miR-148b-3p, hsa-miR-150-5p, hsa-miR-17-5p, hsa-miR-181d-5p, hsa-miR-186-3p, hsa-miR-186-5p, hsa-miR-18a-3p, hsa-miR-1913, hsa-miR-193b-3p, hsa-miR-1976, hsa-miR-204-5p, hsa-miR-205-5p, hsa-miR-206, hsa-miR-20a-5p, hsa-miR-20b-5p, hsa-miR-211-5p, hsa-miR-212-5p, hsa-miR-218-5p, hsa-miR-22-3p, hsa-miR-24-3p, hsa-miR-26b-5p, hsa-miR-300, hsa-miR-302c-3p, hsa-miR-324-3p, hsa-miR-328-3p, hsa-miR-329-3p, hsa-miR-340-5p, hsa-miR-34a-5p, hsa-miR-362-3p, hsa-miR-373-3p, hsa-miR-381-3p, hsa-miR-410-3p, hsa-miR-4275, hsa-miR-4284, hsa-miR-4324, hsa-miR-4460, hsa-miR-4532, hsa-miR-4638-5p, hsa-miR-4673, hsa-miR-4691-5p, hsa-miR-4707-3p, hsa-miR-4727-3p, hsa-miR-4793-5p, hsa-miR-495-3p, hsa-miR-505-3p, hsa-miR-512-3p, hsa-miR-5193, hsa-miR-5196-3p, hsa-miR-520a-3p, hsa-miR-520d-3p, hsa-miR-520h, hsa-miR-539-3p, hsa-miR-582-5p, hsa-miR-652-3p, hsa-miR-660-3p, hsa-miR-665, hsa-miR-92a-3p, and hsa-miR-93-5p.

In the HMGB1-related miRNA group, in addition to miR-142-3p, miR-22-3p, and hsa-miR-129-5p described above, the following specific miRNAs are known to be capable of ameliorating vasculitis, and therefore are also effective as a prophylactic agent or therapeutic agent for acute lung injury, acute respiratory distress syndrome, or sepsis.

### (1-1) In relation to hsa-let-7b-5p

When let-7a and let-7b are overexpressed, endothelial cell apoptosis caused by oxLDL, NO deficiency, reactive oxygen overproduction, increase in LOX-1 expression, and down regulation of endothelial nitric oxide synthase (eNOS) are suppressed, and let-7a and let-7b have a protective effect against endothelial cell injury by oxidized low density lipoprotein (Plos one (2014)9, (9): e106540).

Secreted exosomal miRs (miR-210, miR-23a-3p, miR-424, let-7f, miR-30b, miR-30c, miR-126, miR-21, miR-132, miR-130a-3p, miR-214, miR-378, miR-126, miR-133, and let-7b-5p) induce cardiac angiogenesis and revascularization to increase the blood flow to ischemic myocardium and thereby prevent myocardial ischemia (Heart Failure Reviews, (2021) 26(1), 205-213).

### (1-2) In relation to hsa-miR-100-5p

Since miR-100-5p concentrated into husMSC-exo suppressed FOXO3 expression to suppress activation of NLRP3 inflammasome and suppress cytokine release, and thus could protect cardiomyocytes from H/R-induced pyroptosis and damage (Front. Bioeng. Biotechnol. (2021)8, 615850).

Expression of FZD5 is negatively controlled by miR-100-5p targeting FZD5. Cell proliferation and inflammatory response were suppressed as hUCMSC-Ex-miR-100-5p controls FZD5 of eosinophils. Cell proliferation of eosinophils and inflammatory response via Wnt/β-catenin pathway of eosinophils were suppressed by hUCMSC-Ex-miR-100-5p. The atherosclerotic plaque area and inflammation in mice were reduced by hUCMSC-Ex-miR-100-5p (Acta. Biochim. Biophys. sin. (2021)53 (9): 1166-1176).

### (1-3) In relation to hsa-miR-92a-3p

The target of miR-92a-3p is SIRT6. Knockdown of miR-92a-3p facilitates SIRT6 expression and deactivates MAPK signaling pathway. When SIRT6/MAPK signaling pathway is regulated by miR-92a-3p, apoptosis of HUVECs due to ox-LDL is facilitated (Brazilian Journal of Medical and Biological Research (2021)54 (3): e9386).

It is inferred that, in a case of vascular injury, a proatheroma stimulation promotes release of EMV including miR-92a-3p as a messenger carrying a regenerable signal, which is further taken up by downstream target cells in the blood to promote angiogenesis (Circ. Res. (2019); 124: 575-587).

Forced overexpression of miR-92a in endothelial cells inhibited angiogenesis both in vitro and in vivo. In mouse models of limb ischemia and myocardial infarction, systemic administration of an antagomir designed to inhibit miR-92a promoted vascular growth and led to functional recovery of the damaged tissues (Science (2009)324(5935): 1710-1713).

Inhibition of miR-92a increases endothelial cell proliferation and migration in vitro and suppresses neointimal growth after vascular injury in vivo (Basic Res. Cardiol. (2012)107: 296).

### (1-4) In relation to hsa-miR-34a-5p

Inhibition of Sirt1 by miR-34a induces senescence and inhibits angiogenesis mediated by endothelial progenitor cells (EPCs) is inhibited (Am. J. Physiol. Endocrinol. Metab. (2010)299: E110-E116).

Inhibition of miR-34a suppresses age-related cardiomyocyte death and functional decline in vivo. Induction of miR-34a is caused after acute myocardial infarction, and inhibition thereof promotes the recovery of cardiac contractility following acute myocardial infarction (Nature (2013)495, 107-110).

In the present invention, when the small extracellular vesicles include the miRNA targeting a gene (preferably HMGB1 gene) involved in HMGB1 expression, the small extracellular vesicles preferably function as an expression regulator for a gene involved in HMGB1 expression. The small extracellular vesicles are more preferably an HMGB1 expression suppressant.

In this case, in the present invention, the small extracellular vesicles preferably include at least one of hsa-let-7b-5p, hsa-miR-100-5p, hsa-miR-129-5p, hsa-miR-142-3p, hsa-miR-22-3p, hsa-miR-34a-5p, and hsa-miR-92a-3p, more preferably include at least hsa-let-7b-5p, especially preferably include hsa-let-7b-5p, hsa-miR-100-5p, and hsa-miR-92a-3p, and more especially preferably include all of hsa-let-7b-5p, hsa-miR-100-5p, hsa-miR-129-5p, hsa-miR-142-3p, hsa-miR-22-3p, hsa-miR-34a-5p, and hsa-miR-92a-3p.

The small extracellular vesicles include preferably 2.0 or more, more preferably 4.0 or more, and especially preferably 6.0 or more of at least one of miRNAs targeting a gene involved in HMGB1 expression, in terms of log2ratio of read counts obtained through IMOTA analysis. The small extracellular vesicles include preferably 4.0 or more, more preferably 10.0 or more, especially preferably 12.0 or more, and more especially preferably 15.0 or more of each of hsa-let-7b-5p, hsa-miR-100-5p, hsa-miR-129-5p, hsa-miR-142-3p, hsa-miR-22-3p, hsa-miR-34a-5p, and hsa-miR-92a-3p, independently, in terms of log2ratio of read counts obtained through IMOTA analysis. It is more preferable that 12.0 or more of each of hsa-let-7b-5p, hsa-miR-100-5p, and hsa-miR-92a-3p be included.

The expression amount of hsa-let-7b-5p in the small extracellular vesicles is preferably 1.1 times greater or more, more preferably 1.5 times greater or more, and especially preferably 2 times greater or more compared to exosomes obtained from a culture supernatant of adipose-derived stem cells or exosomes obtained from a culture supernatant of umbilical cord-derived stem cells.

The HMGB1 expression suppressant preferably suppresses HMGB1 gene expression in arbitrary cells by 0.8 times or less, more preferably by 0.6 times or less, and especially preferably by 0.55 times or less of the normal level (in the case of untreated cells).

### Kinds of miRNA

The small extracellular vesicles derived from the culture supernatant of dental pulp-derived stem cells include about 2600 kinds of small RNAs. Among them, about 1800 kinds are miRNAs. Among these miRNAs, miRNAs included in a large content are 180 to 200 kinds. The miRNAs included in the small extracellular vesicles derived from dental pulp-derived stem cells in a large content are characterized in that many of the microRNA are related to treatment of cranial nerve diseases or vasculitis, have not been known heretofore, and are newly found by the present inventor. This characteristic is significantly different from the kinds of miRNAs included in a large content in small extracellular vesicles of other mesenchymal stem cells. For example, miRNAs included in small extracellular vesicles of adipose-derived stem cells or in small extracellular vesicles of umbilical cord-derived stem cells in a large content hardly include microRNAs related to treatment of cranial nerve diseases or vasculitis.

The small extracellular vesicles preferably include two or more kinds, more preferably 10 or more kinds, more preferably 30 or more kinds, especially preferably 50 or more kinds, and more especially preferably 100 or more kinds as the miRNA targeting a gene involved in HMGB1 expression.

### Kinds of small extracellular vesicles

The small extracellular vesicles are preferably at least one kind selected from the group consisting of exosomes, microvesicles, membrane particles, membrane vesicles, ectosomes, and exovesicles, and a microvesicles, and are more preferably exosomes.

The diameter of the small extracellular vesicles is preferably 10 to 1000 nm, more preferably 30 to 500 nm, and especially preferably 50 to 150 nm.

In addition, a molecule known as a tetraspanin such as CD9, CD63, or CD81 is desirably present on surfaces of the small extracellular vesicles, and the molecule may be CD9 alone, CD63 alone, or CD81 alone, or any combination of two or three thereof.

A preferred mode in the case of using exosomes as the small extracellular vesicles will be described below, but the small extracellular vesicles used in the present invention are not limited to exosomes.

The exosomes are preferably extracellular vesicles that are released from a cell when a multivesicular body is fused with a plasma membrane.

Surfaces of the exosomes preferably includes a lipid and a protein derived from a cell membrane of a dental pulp-derived stem cell.

An intracellular substance of the dental pulp-derived stem cell such as a nucleic acid (microRNA, messenger RNA, DNA, or the like) and a protein is preferably included in the inside of the exosomes.

Exosomes are known to be used for cell-to-cell communication through transportation of genetic information from one cell to another cell. Exosomes can be easily tracked and targeted to a specific region.

### Content of small extracellular vesicles

The content of the small extracellular vesicles in the small extracellular vesicle composition is not particularly limited. The small extracellular vesicle composition preferably includes 0.5 × 10⁸ or more small extracellular vesicles, more preferably 1.0 × 10⁸ or more small extracellular vesicles, especially preferably 2.0 × 10⁸ or more small extracellular vesicles, more especially preferably 2.5 × 10⁸ or more small extracellular vesicles, and still more especially preferably 1.0 × 10⁹ or more small extracellular vesicles.

The concentration of the small extracellular vesicles contained in the small extracellular vesicle composition is not particularly limited. The small extracellular vesicle composition preferably includes 1.0 × 10⁸ small extracellular vesicles or more per 1 mL, more preferably 2.0 × 10⁸ small extracellular vesicles or more per 1 mL, especially preferably 4.0 × 10⁸ small extracellular vesicles or more per 1 mL, more especially preferably 5.0 × 10⁸ small extracellular vesicles or more per 1 mL, and still more especially preferably 2.0 × 10⁹ small extracellular vesicles or more per 1 mL.

In a preferred embodiment of the HMGB1 expression regulator of the present invention, the amount of the miRNA targeting a gene involved in HMGB1 expression can be maintained high when the small extracellular vesicles are included in such a large amount or high concentration.

### Other components

The small extracellular vesicle composition may contain, in addition to the small extracellular vesicles, other components according to the purpose and the kind of a subject animal for administration, as long as the effect of the present invention is not impaired. Other components include a nutritional ingredient, an antibacterial agent, a cytokine, a protective agent, a carrier, an excipient, a disintegrant, a buffer, an emulsifier, a suspending agent, a soothing agent, a stabilizer, a preservative, an antiseptic, and the like.

Examples of the nutritional ingredient include a fatty acid and a vitamin.

Examples of the antibacterial agent include penicillin, streptomycin, and gentamicin.

Examples of the carrier can include a known material as a pharmaceutically acceptable carrier.

The small extracellular vesicle composition may be the culture supernatant of dental pulp-derived stem cells itself or the small extracellular vesicles themselves, and may be a pharmaceutical composition further including a pharmaceutically acceptable carrier, excipient, or the like. The purpose of the pharmaceutical composition is to facilitate administration of the small extracellular vesicles to a subject for administration.

The pharmaceutically acceptable carrier is preferably a carrier (including a diluent) which does not cause significant irritation to the subject for administration and which does not inhibit biological activity and properties of the compound to be administered. Examples of the carrier include propylene glycol; (physiological) saline; an emulsion; a buffer solution; a medium such as DMEM or RPMI; and a low-temperature preservation medium containing a component to remove free radicals.

The small extracellular vesicle composition may include an active ingredient of a conventionally known therapeutic agent for acute lung injury, acute respiratory distress syndrome, or sepsis. Those skilled in the art can appropriately change the composition according to the application and subject for administration.

On the other hand, the small extracellular vesicle composition is preferably free from a predetermined substance.

For example, the small extracellular vesicle composition is preferably free from dental pulp-derived stem cells.

In addition, the small extracellular vesicle composition is preferably free from MCP-1. Provided that the small extracellular vesicle composition may include a cytokine other than MCP-1. Additional cytokines include those described in paragraphs [0014] to [0020] of JP2018-023343A.

In addition, the small extracellular vesicle composition is preferably free from Siglec-9. Provided that the small extracellular vesicle composition may include a sialic acid-binding immunoglobulin-like lectin other than Siglec-9.

Note that it is preferable that the small extracellular vesicle composition be substantially free from serum (fetal bovine serum, human serum, sheep serum, and the like). It is also preferable that the small extracellular vesicle composition be substantially free from conventional serum substitutes such as Knockout serum replacement(KSR).

The content (solid content) of each of the other components in the small extracellular vesicle composition is preferably 1% by mass or less, more preferably 0.1% by mass or less, and especially preferably 0.01% by mass or less.

### Method of producing small extracellular vesicle

A method of producing the small extracellular vesicles is not particularly limited.

The HMGB1 expression regulator of the present invention may be prepared by preparing a culture supernatant of dental pulp-derived stem cells or the like, and subsequently purifying small extracellular vesicles from the culture supernatant of dental pulp-derived stem cells. Alternatively, the HMGB1 expression regulator of the present invention may be prepared by purifying small extracellular vesicles from a commercially purchased culture supernatant of dental pulp-derived stem cells. Furthermore, the HMGB1 expression regulator of the present invention may be prepared by taking over a composition including a culture supernatant of dental pulp-derived stem cells that has been discarded (or purifying the composition as appropriate), and purifying small extracellular vesicles therefrom.

### Method of preparing culture supernatant of dental pulp-derived stem cells or the like

The culture supernatant of dental pulp-derived stem cells or the like is not particularly limited.

It is preferable that the culture supernatant of dental pulp-derived stem cells or the like be substantially free from serum. For example, the content of serum in the culture supernatant of dental pulp-derived stem cells or the like is preferably 1% by mass or less, more preferably 0.1% by mass or less, and especially preferably 0.01% by mass or less.

The dental pulp-derived stem cells may be derived from a human or a non-human animal. Animals other than humans can include the same animals as the animals (biological species) to which the HMGB1 expression regulator of the present invention is administered described later, and a mammal is preferable.

The dental pulp-derived stem cells used in the culture supernatant are not particularly limited. Stem cells from exfoliated deciduous teeth, deciduous tooth dental pulp stem cells obtained by another method, and permanent tooth dental pulp stem cells (DPSC) can be used. In addition to human deciduous tooth dental pulp stem cells and human permanent tooth dental pulp stem cells, dental pulp-derived stem cells derived from an animal other than humans such as porcine deciduous tooth dental pulp stem cells can be used.

In addition to exosomes, dental pulp-derived stem cells may produce various cytokines such as a vascular endothelial growth factor (VEGF), a hepatocyte growth factor (HGF), an insulin-like growth factor (IGF), a platelet-derived growth factor (PDGF), transforming growth factor-beta (TGF-β)-1 and -3, TGF-α, KGF, HBEGF, SPARC, other growth factors, and chemokines. Many other physiologically active substances may also be produced.

In the present invention, it is especially preferable that the dental pulp-derived stem cells used in the culture supernatant of dental pulp-derived stem cells be dental pulp-derived stem cells including many proteins, and it is preferable to use deciduous tooth dental pulp stem cells. That is, in the present invention, it is preferable to use a culture supernatant of deciduous tooth dental pulp stem cells.

The dental pulp-derived stem cells used in the present invention may be naturally occurring cells or genetically modified cells as long as an intended treatment can be achieved.

In particular, in the present invention, immortalized stem cells of dental pulp-derived stem cells can be used. By using immortalized stem cells capable of substantially infinite proliferation, the amount and composition of biological factors included in the culture supernatant of stem cells can be stabilized over a long period of time. The immortalized stem cells of dental pulp-derived stem cells are not particularly limited. The immortalized stem cells are preferably non-cancerous immortalized stem cells. The immortalized stem cells of dental pulp-derived stem cells can be prepared by adding one or a combination of the following low-molecular compounds (inhibitors) to the dental pulp-derived stem cells and culturing same.

TGFβ receptor inhibitors are not particularly limited as long as the inhibitors have an effect of inhibiting the function of transforming growth factor (TGF)β receptors, and examples thereof include 2-(5-benzo[1,3]dioxol-4-yl-2-tert-butyl-1H-imidazol-4-yl)-6-methylpyridine, 3-(6-methylpyridin-2-yl)-4-(4-quinolyl)-1-phenylthiocarbamoyl-1H-pyrazole (A-83-01), 2-[(5-chloro-2-fluorophenyl)pteridin-4-yl]pyridin-4-ylamine (SD-208), 3-[(pyridin-2-yl)-4-(4-quinonyl)]-1H-pyrazole, 2-(3-(6-methylpyridin-2-yl)-1H-pyrazol-4-yl)-1,5-naphthyridine (all of which are from Merck KGaA), and SB431542 (Sigma-Aldrich Corporation). A preferred example is A-83-01.

ROCK inhibitors are not particularly limited as long as the inhibitors have an effect of inhibiting the function of Rho-associated kinase. Examples of the ROCK inhibitors include GSK269962A (Axon Medchem BV), Fasudil hydrochloride (Tocris Bioscience), and Y-27632 and H-1152 (both of which are from FUJIFILM Wako Pure Chemical Corporation). A preferred example is Y-27632.

GSK3 inhibitors are not particularly limited as long as the inhibitors inhibit glycogen synthase kinase 3 (GSK-3), and examples thereof include A1070722, BIO, and BIO-acetoxime (all of which are from TOCRIS corporation).

MEK inhibitors are not particularly limited as long as the inhibitors have an effect of inhibiting the function of MAP kinase-ERK kinase (MEK), and examples thereof include AZD6244, CI-1040(PD184352), PD0325901, RDEA119 (BAY86-9766), SL327, and U0126-EtOH (all of which are from Selleck corporation), and PD98059, U0124 and U0125 (all of which are from Cosmo Bio Co. Ltd.).

When the HMGB1 expression regulator of the present invention is used in regenerative medicine, the culture supernatant of dental pulp-derived stem cells or immortalized stem cells thereof, or the composition including small extracellular vesicles derived therefrom is made into a form that does not contain other somatic stem cells other than dental pulp-derived stem cells or the like, in line with the request under the Act on the Safety of Regenerative Medicine. The small extracellular vesicle composition may contain mesenchymal stem cells or other somatic stem cells other than dental pulp-derived stem cells or the like, but preferably does not contain stem cells other than dental pulp-derived stem cells or the like.

Examples of somatic stem cells other than mesenchymal stem cells include, but are not limited to, stem cells derived from the dermal system, digestive system, myeloid system, nervous system, and the like. Examples of somatic stem cells of the dermal system include epithelial stem cells and hair follicle stem cells. Examples of somatic stem cells of the digestive system include pancreatic (general) stem cells and liver stem cells. Examples of somatic stem cells of the myeloid system (other than mesenchymal stem cells) include hematopoietic stem cells. Examples of somatic stem cells of the nervous system include neural stem cells and retinal stem cells.

The small extracellular vesicle composition may contain stem cells other than somatic stem cells, but preferably does not contain stem cells other than somatic stem cells. Stem cells other than somatic stem cells include embryonic stem cells (ES cells), induced pluripotent stem cells (iPS cells), and embryonic carcinoma cells (EC cells).

A method of preparing the culture supernatant of dental pulp-derived stem cells or immortalized stem cells thereof is not particularly limited, and a conventional method can be used.

The culture supernatant of dental pulp-derived stem cells or the like is a culture solution obtained by culturing dental pulp-derived stem cells. For example, a culture supernatant usable in the present invention can be obtained by separating and removing cell components after culturing dental pulp-derived stem cells. A culture supernatant appropriately subjected to various treatments (for example, centrifugation, concentration, solvent replacement, dialysis, freezing, drying, lyophilization, dilution, and desalination, storage) may be used.

The dental pulp-derived stem cells for obtaining the culture supernatant of dental pulp-derived stem cells can be selected by a conventional method, and can be selected on the basis of the size or morphology of the cells or can be selected as adherent cells. Cells can be selected as adherent cells or passaged cells thereof from dental pulp cells collected from a shed deciduous tooth or a permanent tooth. The culture supernatant obtained by culturing the selected stem cells can be used as the culture supernatant of dental pulp-derived stem cells.

The "culture supernatant of dental pulp-derived stem cells or the like" is preferably a culture solution including no cells directly obtained by culturing dental pulp-derived stem cells or the like. In one embodiment, the culture supernatant of dental pulp-derived stem cells used in the present invention is preferably free from cells (regardless of the type of cells) as a whole. The composition of this embodiment is clearly distinguished by this characteristic not only from the dental pulp-derived stem cells themselves but also from various compositions including dental pulp-derived stem cells. A typical example of this embodiment is a composition that does not include dental pulp-derived stem cells and is composed only of the culture supernatant of dental pulp-derived stem cells.

The culture supernatant of dental pulp-derived stem cells used in the present invention may include a culture supernatant of both deciduous tooth dental pulp-derived stem cells and adult dental pulp-derived stem cells. The culture supernatant of dental pulp-derived stem cells used in the present invention preferably includes, as an active ingredient, the culture supernatant of deciduous tooth dental pulp-derived stem cells, and the content thereof is more preferably 50% by mass or more and preferably 90% by mass or more. It is more especially preferable that the culture supernatant of dental pulp-derived stem cells used in the present invention be a composition composed only of the culture supernatant of deciduous tooth dental pulp-derived stem cells.

A basal medium, a medium obtained by adding serum or the like to a basal medium, or the like can be used as the culture solution of dental pulp-derived stem cells for obtaining the culture supernatant. Iscove's Modified Dulbecco's Medium (IMDM) (GIBCO or the like), Ham's F12 medium (HamF12) (SIGMA, GIBCO, or the like), RPMI1640 medium or the like can be used as the basal medium in addition to Dulbecco's Modified Eagle Medium (DMEM). Examples of a component that can be added to the medium can include serum (fetal bovine serum, human serum, sheep serum, or the like), a serum substitute (Knockout serum replacement(KSR) and the like), bovine serum albumin (BSA), an antibacterial agent, various vitamins, and various minerals.

However, in order to prepare a "culture supernatant of dental pulp-derived stem cells" free from serum, a medium free from serum may be used throughout the entire process or in the last or the last few passages of passage culture. For example, a culture supernatant of dental pulp-derived stem cells free from serum can be prepared by culturing dental pulp-derived stem cells in a medium free from serum (serum-free medium). The culture supernatant of dental pulp-derived stem cells or the like free from serum can also be obtained by performing one or more passages of passage culture, with the last or the last few passages cultured in a serum-free medium. On the other hand, the culture supernatant of dental pulp-derived stem cells free from serum can also be obtained by removing serum from a collected culture supernatant using dialysis, solvent replacement with a column, or the like.

Conditions usually used can be directly applied to cultivation of dental pulp-derived stem cells to obtain the culture supernatant. A method of preparing the culture supernatant of dental pulp-derived stem cells may be the same as the cell culturing method described later, except that the steps of isolating and selecting stem cells are appropriately adjusted according to the type of stem cells. Those skilled in the art can appropriately isolate and select dental pulp-derived stem cells according to the type of the dental pulp-derived stem cells.

In addition, a special condition may be applied to the cultivation of dental pulp-derived stem cells in order to produce a large amount of small extracellular vesicles such as exosomes. Examples of the special condition can include a condition of co-culturing with a certain kind of stimulus such as a low temperature condition, a low oxygen condition, or a microgravity condition.

The culture supernatant of dental pulp-derived stem cells used in the preparation of the small extracellular vesicles such as exosomes in the present invention may include other components in addition to the culture supernatant of dental pulp-derived stem cells, but is preferably substantially free from other components.

However, each kind of additive used in the preparation of exosomes may be added to the culture supernatant of dental pulp-derived stem cells and then stored.

### Preparation of small extracellular vesicles

The small extracellular vesicles can be prepared by purifying small extracellular vesicles from the culture supernatants of dental pulp-derived stem cells or the like.

The purification of the small extracellular vesicles is preferably separation of a fraction including small extracellular vesicles from the culture supernatant of dental pulp-derived stem cells, and more preferably isolation of small extracellular vesicles.

The small extracellular vesicles may be isolated through separation from non-associated components on the basis of the properties of the small extracellular vesicles. For example, the small extracellular vesicles may be isolated on the basis of the molecular weight, size, form, composition, or biological activity.

In the present invention, the small extracellular vesicles can be purified by partitioning a certain fraction (for example, precipitate) including a large number of small extracellular vesicles obtained by centrifuging the culture supernatant of dental pulp-derived stem cells. Unnecessary components (insoluble components) of fractions other than the certain fraction may be removed. The removal of solvents and dispersion media, as well as the unnecessary components, from the small extracellular vesicle composition may not be complete removal. Conditions of centrifugation are, for example, 100 to 20000 g for 1 to 30 minutes.

In the present invention, the small extracellular vesicles can be purified by subjecting the culture supernatant of dental pulp-derived stem cells or a centrifuged product thereof to filtration. Unnecessary components can be removed through the filtration. When a filtration membrane having an appropriate pore size is used, removal of unnecessary components and sterilization can be simultaneously carried out. The material, pore size, and the like of the filtration membrane used in the filtration are not particularly limited. Filtration can be carried out by a known method using a filtration membrane with an appropriate molecular weight or size cut-off. The pore size of the filtration membrane is preferably 10 to 1000 nm, more preferably 30 to 500 nm, and especially preferably 50 to 150 nm, from the viewpoint of easily fractioning exosomes.

In the present invention, the culture supernatant of dental pulp-derived stem cells, a centrifuged product thereof, or a filtered product thereof can be separated using an additional separation means such as RAM chromatography. For example, high performance liquid chromatography (HPLC) using various columns can be used. A size exclusion column or an affinity column can be used as the columns.

One or more properties or biological activities of the small extracellular vesicles can be utilized to track small extracellular vesicles (or activities thereof) in the respective fractions in each treatment step. For example, a light scattering, refractive index, dynamic light scattering, or UV-visible light detector can be used to track small extracellular vesicles. Alternatively, a specific enzymatic activity and the like can be used to track activities in each fraction.

The method described in paragraphs [0034] to [0064] of JP2019-524824A may be used as a method of purifying small extracellular vesicles, and the content of this publication is incorporated herein by reference.

The final form of the small extracellular vesicle composition is not particularly limited. For example, the small extracellular vesicle composition has a form in which the small extracellular vesicles fill a container together with a solvent or a dispersion medium; a form in which the small extracellular vesicles are formed into gel together with gel and fill a container; and a form in which the small extracellular vesicles are frozen and/or dried and solidified and are then prepared as a formulation or fill a container. Examples of the container include a tube, a centrifuge tube, and a bag suitable for cryopreservation. The freezing temperature may be, for example, -20°C to -196°C.

The HMGB1 expression regulator of the present invention has the following advantages: the HMGB1 expression regulator is easily produced in mass quantities as compared with a conventional composition which can be used as a therapeutic agent or a prophylactic agent for acute lung injury, acute respiratory distress syndrome, or sepsis; a culture solution of stem cells which has conventionally been discarded as industrial waste or the like can be utilized; costs of discarding culture solutions of stem cells can be reduced; and the like. In particular, in a case where the culture supernatant of dental pulp-derived stem cells is a culture supernatant of human dental pulp-derived stem cells, when the HMGB1 expression regulator of the present invention is applied to humans, there are advantages of high safety in terms of immunology and the like and fewer ethical issues. When the culture supernatant of dental pulp-derived stem cells is a culture supernatant of dental pulp-derived stem cells from a patient with acute lung injury, acute respiratory distress syndrome, or sepsis, safety of application of the HMGB1 expression regulator of the present invention to the patient will be enhanced, and ethical issues will decrease.

When the HMGB1 expression regulator of the present invention is derived from a culture supernatant of dental pulp-derived stem cells, the HMGB1 expression regulator is also used in reparative medicine. In particular, a composition including small extracellular vesicles derived from a culture supernatant of dental pulp-derived stem cells or the like is preferably used in reparative medicine application. Here, in regenerative medicine on premise for stem cell transplantation, it is known that stem cells do not play a main role in regeneration, and a humoral component produced by stem cells repairs an organ together with self-stem cells. Difficult problems associated with conventional stem cell transplantation such as canceration, standardization, administration methods, preservability, and culture methods are solved, and reparative medicine becomes possible with a composition using a culture supernatant of dental pulp-derived stem cells or small extracellular vesicles derived therefrom. As compared with stem cell transplantation, when the HMGB1 expression regulator of the present invention is used, tumorigenesis or the like is less likely to occur since cells are not transplanted, which may be said to be safer. In addition, the HMGB1 expression regulator of the present invention has the advantage of enabling use of a standardized product with consistent quality. Since mass production and an efficient administration method can be selected, the HMGB1 expression regulator of the present invention can be used at a low cost.

### Prophylactic agent or therapeutic agent for acute lung injury, acute respiratory distress syndrome or sepsis

A prophylactic agent or a therapeutic agent for acute lung injury, acute respiratory distress syndrome, or sepsis of the present invention includes, as an active ingredient, the HMGB1 expression regulator of the present invention.

In the present specification, the term "prophylaxis" refers to precritical prevention of a disease (acute lung injury, acute respiratory distress syndrome, or sepsis in the present specification). In addition, in the present specification, the term "therapy" refers to alleviation, suppression, or inhibition of progression of symptoms of a developed disease and amelioration of symptoms.

### Method of ameliorating acute lung injury, acute respiratory distress syndrome, or sepsis

A method of ameliorating acute lung injury, acute respiratory distress syndrome, or sepsis according to the present invention includes administrating an effective amount of the HMGB1 expression regulator of the present invention or administrating an effective amount of the prophylactic agent or the therapeutic agent for acute lung injury, acute respiratory distress syndrome, or sepsis of the present invention to a subject developing acute lung injury, acute respiratory distress syndrome, or sepsis.

The step of administering the HMGB1 expression regulator or the like of the present invention to a subject developing acute lung injury, acute respiratory distress syndrome, or sepsis is not particularly limited.

Examples of administration methods include spraying or inhalation into the oral cavity, nasal cavity, or respiratory tract, instillation, topical administration, and rhinenchysis, with less invasiveness being preferred. As a method of topical administration, injection is preferable. In addition, electroporation in which fine holes are temporarily formed in cell membranes by applying a voltage (electric pulse) to the skin surface, and the active ingredient is allowed to penetrate into the dermis layer which is not reached through ordinary care is also preferable. Examples of the topical administration include intravenous administration, intraarterial administration, intraportal administration, intradermal administration, subcutaneous administration, intramuscular administration, and intraperitoneal administration, and intraarterial administration, intravenous administration, subcutaneous administration, and intraperitoneal administration are more preferable.

A variety of formulation techniques may also be used to alter the in vivo distribution of the small extracellular vesicles. Numerous methods of altering the in vivo distribution are known to those skilled in the art. Examples of such methods include protection of exosomes in vesicles composed of a material such as a protein, a lipid (for example, a liposome), a carbohydrate, or a synthetic polymer.

The HMGB1 expression regulator of the present invention administered to a subject developing acute lung injury, acute respiratory distress syndrome, or sepsis may circulate in the body of the subject and reach a predetermined tissue.

A frequency of administration and an administration interval are not particularly limited. The frequency of administration can be 1 or more, preferably 5 or more, more preferably 6 or more, and especially preferably 7 or more per week. The administration interval is preferably 1 hour to 1 week, more preferably half a day to 1 week, and especially preferably 1 day (once daily). However, the frequency of administration and the administration interval can be appropriately adjusted according to the species of the subject for administration and symptoms of the subject for administration.

The HMGB1 expression regulator of the present invention is preferably used to administer the small extracellular vesicles once or more times a week over a therapeutically effective period to a subject developing acute lung injury, acute respiratory distress syndrome, or sepsis. When the subject for administration is a human, the frequency of administration per week is preferably high, preferably 5 times or more per week over a therapeutically effective period, and preferably daily administrations.

When a culture supernatant of dental pulp-derived stem cells at a concentration of 2.0 × 10⁹ small extracellular vesicles per 1 ml is used, in a case of a mouse model, 0.1 to 5 ml is preferable, 0.3 to 3 ml is more preferable, and 0.5 to 1 ml is more especially preferable per mouse (about 25 g).

When the small extracellular vesicles are used at a concentration of 0.1 × 10⁸ small extracellular vesicles per 1 µg, in a case of the mouse model, 1 to 50 µg is preferable, 3 to 30 µg is more preferable, and 5 to 25 µg is more especially preferable per mouse (about 25 g).

A preferred range of dosages per body weight for other animals can be calculated using a proportional relationship from dosages per body weight (about 25 g) to the model mice. However, the range of dosages per body weight can be appropriately adjusted according to the symptoms of the subject for administration.

Subject animals (organism species) to which the HMGB1 expression regulator of the present invention is administered are not particularly limited. The subject animals to which the HMGB1 expression regulator of the present invention is administered are preferably mammals, birds (such as chickens, quails, and ducks), and fish (such as salmon, trout, tuna, and bonito). Mammals may be humans or non-human mammals, but humans are especially preferred. The non-human mammals are more particularly cows, pigs, horses, goats, sheep, monkeys, dogs, cats, mice, rats, guinea pigs, and hamsters are more preferable.

The HMGB1 expression regulator of the present invention may be used in combination with a conventionally known therapeutic agent for acute lung injury, acute respiratory distress syndrome, or sepsis. Specifically, the HMGB1 expression regulator of the present invention may be used in combination with, for example, a conventionally known steroid, an immunosuppressive agent, a biological medicine (tocilizumab, a TNF inhibitor, or the like), an antiplatelet drug, an antihistamine or an antiallergic drug, or NSAIDs (nonsteroidal anti-inflammatory drugs.

### Examples

Hereinafter, the features of the present invention will be described more specifically with reference to the Example and Comparative Example or Reference Examples. The materials, amounts used, ratios, treatments, treatment procedures, and the like shown in the following examples can be appropriately changed without departing from the spirit of the present invention. Therefore, the scope of the present invention should not be construed as being limited by the following specific example.

### Example 1

### Preparation of culture supernatant of dental pulp-derived stem cells

A culture supernatant of human deciduous tooth pulp stem cells was prepared according to the method described in Example 6 of JP6296622B except that a DMEM medium was used instead of the DMEM/HamF12 mixed medium, and the culture supernatant was partitioned. In the primary culture, culture was performed with addition of fetal bovine serum (FBS), and in the passage culture, the supernatant of the passage culture solution cultured using the primary culture solution was partitioned so as not to contain FBS, and a culture supernatant of deciduous tooth pulp stem cells was prepared. Incidentally, DMEM was Dulbecco's Modified Eagle Medium, and F12 was Ham's F12 Medium.

### Preparation of exosomes

Exosomes of dental pulp-derived stem cells were purified from the obtained culture supernatant of dental pulp-derived stem cells by the following method.

The culture supernatant (100 mL) of deciduous tooth pulp stem cells was filtered using a filter having a pore size of 0.22 micrometers, and the obtained solution was centrifuged at 100000×g for 60 minutes at 4°C. The supernatant was decanted, and the exosome-enriched pellet was resuspended in phosphate-buffered saline (PBS). The resuspended sample was centrifuged at 100000×g for 60 minutes. The pellet was collected again as a concentrated sample from the bottom of the centrifuge tube (approximately 100 µl). The protein concentration was determined by micro BCA protein assay kit (Pierce Biotechnology Inc., Rockford, IL). The composition including exosomes (concentrated solution) was stored at -80°C.

The composition including exosomes purified from the culture supernatant of dental pulp-derived stem cells was used as an expression regulator (small extracellular vesicle composition sample) of Example 1.

The average particle diameter and concentration of the small extracellular vesicles included in the expression regulator of Example 1 were evaluated using a nanoparticle analysis system NanoSight (manufactured by Quantum Design Japan, Inc.).

The average particle diameter of the small extracellular vesicles included in the expression regulator of Example 1 was 50 to 150 nm.

The expression regulator of Example 1 was a high concentration exosome solution including 1.0 × 10⁹ small extracellular vesicles or more per 1 ml and specifically a high concentration exosome solution including 2.0 × 10⁹ small extracellular vesicles or more per 1 ml.

The components of the obtained expression regulator of Example 1 were analyzed by a known method. As a result, it was found that the expression regulator of Example 1 did not contain stem cells of dental pulp-derived stem cells, did not contain MCP-1, and did not contain Siglec 9. Therefore, it was found that the active ingredient of the expression regulator of Example 1 was different from MCP-1 and Siglec 9, which are active ingredients of a culture supernatant of mesenchymal stem cells, and their analogs.

### Comparative Example 1

### Preparation of culture supernatant of umbilical cord-derived stem cells

A culture supernatant of umbilical cord-derived stem cells was prepared according to Example 1 except that human umbilical cord-derived stem cells were used instead of deciduous tooth pulp stem cells, and a composition (small extracellular vesicle composition of Comparative Example 1) including exosomes purified from the umbilical cord-derived stem cells was prepared.

### Test Example 1: microRNAs expressed by exosomes

The small RNAs included in the small extracellular vesicle composition of Example 1 were analyzed by next generation sequencing (NGS) analysis. NGS analysis identified 1787 miRNAs included in the small extracellular vesicle composition of Example 1 (exosomes of dental pulp-derived stem cells). The obtained results are shown in Table 1 below.

**[Table 1]**

| Name | Detected number | % |
|---|---|---|
| miRNAs | 1787 | 72.35 |
| piRNAs | 123 | 4.98 |
| tRNAs | 411 | 16.64 |
| Other RNAs | 149 | 6.03 |
| Total number | 2470 | 100 |

### Test Example 2: search for microRNAs associated with disease

Disease-associated microRNAs were searched for. Interactive Multi-Omics-Tissue Atlas (IMOTA) was used for extraction of disease-associated miRNAs. IMOTA is an interactive multi-omics atlas capable of examining the interaction and expression levels of miRNAs, mRNAs, and proteins in each tissue or cell (Nucleic Acids Research, Volume 46, Issue D1, 4 January 2018, Pages D770-D775, "IMOTA: an interactive multi-omics tissue atlas for the analysis of human miRNA-target interactions"). Here, it was investigated whether microRNAs that regulate a protein and/or a gene involved in acute lung injury or sepsis were included, or whether microRNAs that regulate a protein and/or a gene targeted by a therapeutic agent were included. In Test Example 2, miRNAs targeting a gene involved in HMGB1 expression were searched for.

The miRNAs expressed in the exosomes purified from the culture supernatant of dental pulp-derived stem cells targeting a gene involved in HMGB1 expression were the following HMGB1-related miRNA group (78 kinds).

HMGB1-related miRNA group:
hsa-let-7b-5p, hsa-let-7e-5p, hsa-let-7g-5p, hsa-miR-100-5p, hsa-miR-103a-3p, hsa-miR-106b-5p, hsa-miR-107, hsa-miR-1179, hsa-miR-1183, hsa-miR-1236-3p, hsa-miR-1237-3p, hsa-miR-1247-3p, hsa-miR-129-5p, hsa-miR-1304-3p, hsa-miR-1307-3p, hsa-miR-141-3p, hsa-miR-142-3p, hsa-miR-142-5p, hsa-miR-145-5p, hsa-miR-148a-3p, hsa-miR-148b-3p, hsa-miR-150-5p, hsa-miR-17-5p, hsa-miR-181d-5p, hsa-miR-186-3p, hsa-miR-186-5p, hsa-miR-18a-3p, hsa-miR-1913, hsa-miR-193b-3p, hsa-miR-1976, hsa-miR-204-5p, hsa-miR-205-5p, hsa-miR-206, hsa-miR-20a-5p, hsa-miR-20b-5p, hsa-miR-211-5p, hsa-miR-212-5p, hsa-miR-218-5p, hsa-miR-22-3p, hsa-miR-24-3p, hsa-miR-26b-5p, hsa-miR-300, hsa-miR-302c-3p, hsa-miR-324-3p, hsa-miR-328-3p, hsa-miR-329-3p, hsa-miR-340-5p, hsa-miR-34a-5p, hsa-miR-362-3p, hsa-miR-373-3p, hsa-miR-381-3p, hsa-miR-410-3p, hsa-miR-4275, hsa-miR-4284, hsa-miR-4324, hsa-miR-4460, hsa-miR-4532, hsa-miR-4638-5p, hsa-miR-4673, hsa-miR-4691-5p, hsa-miR-4707-3p, hsa-miR-4727-3p, hsa-miR-4793-5p, hsa-miR-495-3p, hsa-miR-505-3p, hsa-miR-512-3p, hsa-miR-5193, hsa-miR-5196-3p, hsa-miR-520a-3p, hsa-miR-520d-3p, hsa-miR-520h, hsa-miR-539-3p, hsa-miR-582-5p, hsa-miR-652-3p, hsa-miR-660-3p, hsa-miR-665, hsa-miR-92a-3p, and hsa-miR-93-5p.

Therefore, it was found that the expression regulator of the present invention can be used as an HMGB1 expression regulator.

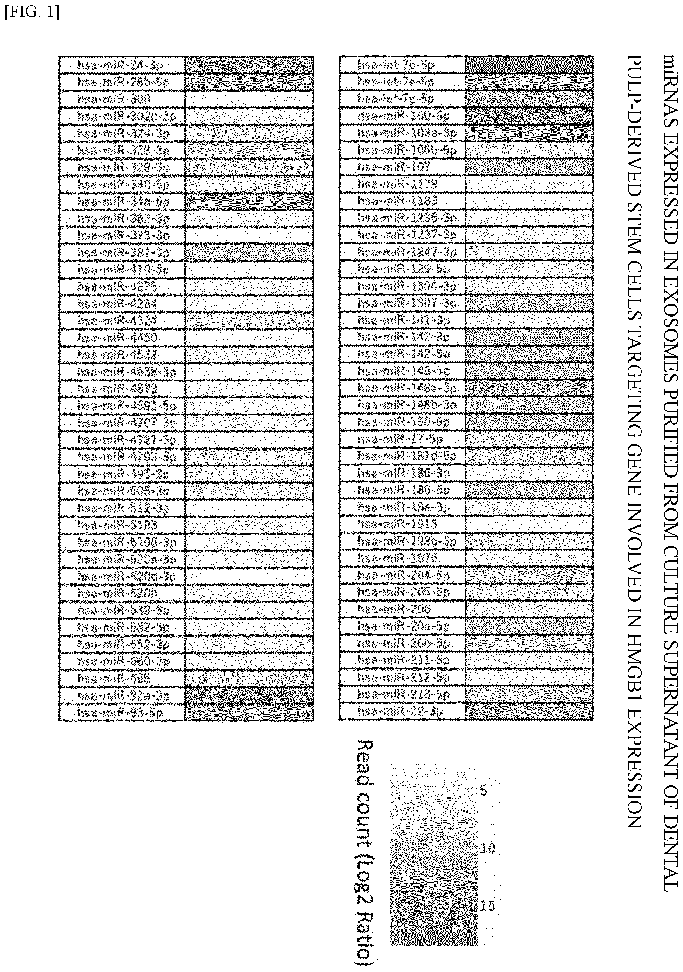

Results of comparing the expression levels of miRNAs expressed in exosomes of dental pulp-derived stem cells are shown in the heat map of FIG. 1. The concentration of the heat map is indicated by log ratio of the read count value.

From FIG. 1, it was found that the expression suppressant of the present invention included miRNAs related to HMGB1 at a high concentration. Therefore, the expression suppressant of the present invention can control the expression of HMGB1 and/or HMGB1 gene (Hmgb1).

### Test Example 3: Confirmation of suppression of HMGB1 expression

Suppression of HMGB1 expression was confirmed by the following method using a vasculitis model of human umbilical vein endothelial cells (HUVECs).

HUVECs were inoculated and then cultured at 37°C under a 5% CO condition for 18 hours. Thereafter, HUVECs were sealed in a BBL GasPakTM anaerobic system (Becton Dickinson Microbiology Systems, Cockeysville, MD, USA) and cultured for 24 hours under low oxygen. Consequently, the culture environment for the HUVECs had an oxygen concentration of 2%.

After 24 hours, the expression regulator (exosomes purified from dental pulp-derived stem cells) of Example 1 or the small extracellular vesicle composition (exosomes purified from umbilical cord-derived stem cells) prepared in Comparative Example 1 was administered as 10000 exosomes per one HUVEC.

After 48 hours, the HMGB1 gene expression level was quantified by qPCR using the following primer set.

HMGB1:
HMG1(HMGB1) Human qPCR Primer Pair (NM _002128)
GCGAAGAAACTGGGAGAGATGTG/GCATCAGGCTTTCCTTTAGCTCG

### Reference Example 1: without low oxygen treatment

The HMGB1 gene expression level was quantified by qPCR in the same manner as in Example 1 except that the treatment of culturing HUVECs under low oxygen for 24 hours was replaced with culturing at 37°C under a 5% CO condition for 24 hours and the small extracellular vesicle composition was not added.

### Reference Example 2: without addition of small extracellular vesicles after low oxygen culturing

The HMGB1 gene expression level was quantified by qPCR in the same manner as in Example 1 except that the small extracellular vesicle composition was not added.

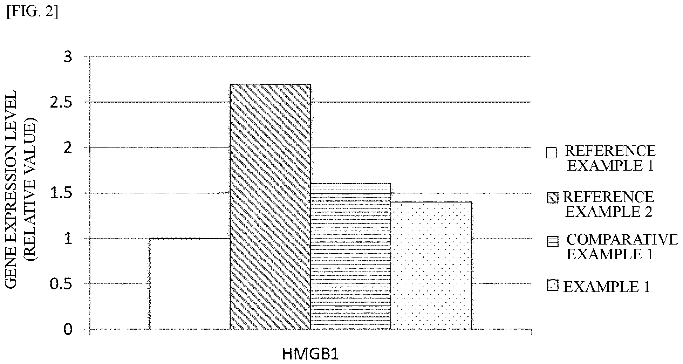

FIG. 2 is a graph of HMGB1 gene expression levels quantified in the respective examples. From FIG. 2, it was found that the HMGB1 expression suppressant of the present invention can significantly suppress the HMGB1 gene expression level.

## Claims

1. An HMGB1 expression regulator,
wherein the HMGB1 expression regulator comprises small extracellular vesicles, and
the small extracellular vesicles include a miRNA targeting a gene involved in HMGB1 expression.

2. The HMGB1 expression regulator according to claim 1, wherein
the small extracellular vesicles are exosomes.

3. The HMGB1 expression regulator according to claim 1, wherein
the miRNA targeting a gene involved in HMGB1 expression includes at least one miRNA in an HMGB1-related miRNA group:
hsa-let-7b-5p, hsa-let-7e-5p, hsa-let-7g-5p, hsa-miR-100-5p, hsa-miR-103a-3p, hsa-miR-106b-5p, hsa-miR-107, hsa-miR-1179, hsa-miR-1183, hsa-miR-1236-3p, hsa-miR-1237-3p, hsa-miR-1247-3p, hsa-miR-129-5p, hsa-miR-1304-3p, hsa-miR-1307-3p, hsa-miR-141-3p, hsa-miR-142-3p, hsa-miR-142-5p, hsa-miR-145-5p, hsa-miR-148a-3p, hsa-miR-148b-3p, hsa-miR-150-5p, hsa-miR-17-5p, hsa-miR-181d-5p, hsa-miR-186-3p, hsa-miR-186-5p, hsa-miR-18a-3p, hsa-miR-1913, hsa-miR-193b-3p, hsa-miR-1976, hsa-miR-204-5p, hsa-miR-205-5p, hsa-miR-206, hsa-miR-20a-5p, hsa-miR-20b-5p, hsa-miR-211-5p, hsa-miR-212-5p, hsa-miR-218-5p, hsa-miR-22-3p, hsa-miR-24-3p, hsa-miR-26b-5p, hsa-miR-300, hsa-miR-302c-3p, hsa-miR-324-3p, hsa-miR-328-3p, hsa-miR-329-3p, hsa-miR-340-5p, hsa-miR-34a-5p, hsa-miR-362-3p, hsa-miR-373-3p, hsa-miR-381-3p, hsa-miR-410-3p, hsa-miR-4275, hsa-miR-4284, hsa-miR-4324, hsa-miR-4460, hsa-miR-4532, hsa-miR-4638-5p, hsa-miR-4673, hsa-miR-4691-5p, hsa-miR-4707-3p, hsa-miR-4727-3p, hsa-miR-4793-5p, hsa-miR-495-3p, hsa-miR-505-3p, hsa-miR-512-3p, hsa-miR-5193, hsa-miR-5196-3p, hsa-miR-520a-3p, hsa-miR-520d-3p, hsa-miR-520h, hsa-miR-539-3p, hsa-miR-582-5p, hsa-miR-652-3p, hsa-miR-660-3p, hsa-miR-665, hsa-miR-92a-3p, and hsa-miR-93-5p.

4. The HMGB1 expression regulator according to claim 1, wherein
the miRNA targeting a gene involved in HMGB1 expression includes at least one of hsa-let-7b-5p, hsa-miR-100-5p, hsa-miR-129-5p, hsa-miR-142-3p, hsa-miR-22-3p, hsa-miR-34a-5p, and hsa-miR-92a-3p.

5. The HMGB1 expression regulator according to claim 1, wherein
the miRNA targeting a gene involved in HMGB1 expression includes at least hsa-let-7b-5p.

6. The HMGB1 expression regulator according to claim 1, wherein
the small extracellular vesicles include the miRNA targeting a gene involved in HMGB1 expression at a concentration higher than a culture supernatant of dental pulp-derived stem cells.

7. The HMGB1 expression regulator according to claim 1, wherein
the small extracellular vesicles are isolated through purification of a culture supernatant of dental pulp-derived stem cells, and
the small extracellular vesicles do not include a component in the culture supernatant of dental pulp-derived stem cells excluding exosomes.

8. A prophylactic agent or therapeutic agent for acute lung injury, acute respiratory distress syndrome, or sepsis, comprising, as an active ingredient, the HMGB1 expression regulator according to claim 1.

9. A method of ameliorating acute lung injury, acute respiratory distress syndrome, or sepsis, the method comprising administrating an effective amount of the HMGB1 expression regulator according to claim 1 or an effective amount of the prophylactic agent or therapeutic agent for acute lung injury, acute respiratory distress syndrome, or sepsis according to claim 8 to a subject developing acute lung injury, acute respiratory distress syndrome, or sepsis.
